# EUROPÄISCHE PATENTSCHRIFT

(11) **EP 3 167 031 B1**
(45) Veröffentlichungstag und Bekanntmachung des Hinweises auf die Patenterteilung: **09.02.2022**
(21) Anmeldenummer: 15734386.4
(22) Anmeldetag: 07.07.2015
(51) Int. Cl.: C11B 9/00, A61Q 13/00, A61L 9/01, C11D 3/50

(54) **ISOMERENMISCHUNGEN VON UNGESÄTTIGTEN MAKROCYCLISCHEN MOSCHUSVERBINDUNGEN**
ISOMERS MIXTURES OF UNSATURATED MACROCYCLIC MUSK COMPOUNDS
MÉLANGES D'ISOMÈRES DE COMPOSÉS MUSQUÉS MACROCYLIQUES INSATURÉS

(30) Priorität: 07.07.2014 EP 14176002
(43) Veröffentlichungstag der Anmeldung: 17.05.2017
(60) Teilanmeldung: 21214477.8
(73) Patentinhaber: Symrise AG, 37603 Holzminden (DE)
(72) Erfinder: HÖLSCHER, Bernd, 37620 Halle (DE); WIEDMANN, Wilhelm, 37639 Bevern (DE); SIEGEL, Sven, 37671 Höxter (DE); KULKE, Torsten, 37671 Höxter-Lüchtringen (DE)
(74) Vertreter: Eisenführ Speiser
(86) Internationale Anmeldenummer: PCT/EP2015/065429
(87) Internationale Veröffentlichungsnummer: WO 2016/005361

(56) Entgegenhaltungen:
- EP-A1- 2 662 098
- EP-A2- 0 818 452
- WO-A1-03/037841
- WO-A1-2005/063670
- US-A- 4 568 470

## Beschreibung

Die vorliegende Erfindung betrifft primär neue Mischungen, enthaltend oder bestehend aus trans- und cis-konfiguriertem Oxacyclohexadecen-2-on, wobei das Gewichtsverhältnis von der Verbindung (E) zu der Verbindung (Z) im Bereich von 10 : 90 bis 60 : 40 liegt, oder aus trans- und cis-konfiguriertem Oxacycloheptadec-10-en-2-on, wobei das Gewichtsverhältnis von der Verbindung (E) zu der Verbindung (Z) im Bereich von 10 : 90 bis 60 : 40 liegt.

Die vorliegende Erfindung betrifft weiterhin parfümierte Produkte enthaltend eine erfindungsgemäße Mischung (wie hierin beschrieben), Verfahren zur Herstellung erfindungsgemäßer Mischungen, Verfahren zur Herstellung erfindungsgemäßer parfümierter Produkte sowie bevorzugte Verwendungen erfindungsgemäßer Mischungen, insbesondere (a) zum Maskieren oder Vermindern des bzw. eines oder mehrerer unangenehmer Geruchseindrücke eines oder mehrerer unangenehm riechender Stoffe, und/oder (b) zum Verstärken des bzw. eines oder mehrerer angenehmer Geruchseindrücke eines oder mehrerer angenehm riechender Stoffe.

Weitere Aspekte und bevorzugte Ausgestaltungen der vorliegenden Erfindung ergeben sich aus der nachfolgenden Beschreibung sowie insbesondere den beigefügten Patentansprüchen.

Grundsätzlich besteht seitens der Parfümindustrie ein ständiger Bedarf, angenehme geruchliche Aspekte von Riechstoffen zu betonen (hervorzuheben / zu verstärken) und unangenehme geruchliche Aspekte zu maskieren oder zu vermindern. Insbesondere die hierin beschriebenen mit erfindungsgemäß zu verwendenden Verbindungen vorteilhafterweise bevorzugt zu kombinierende Riechstoffe spielen in der Parfümerie eine wichtige Rolle. Dabei ist es gewünscht, einerseits deren natürliche Frische und/oder Ausstrahlung besonders hervorzuheben und andererseits neue Effekte zu entwickeln.

Die Offenlegungsschrift EP 0 818 452 A2 offenbart die Synthese von cis-Oxacycloheptadec-11-en-2-on und cis-Oxacycloheptadec-12-en-2-on, Riechstoffkompositionen enthaltend die besagten Produkte und Verfahren zum Vermitteln eines amber- oder moschusartigen Dufts mithilfe der besagten Produkte.

Die Offenlegungsschrift WO 2005/063670 A1 beschreibt die Verbindung (**Z**)-7-Cyclohexadecen-1-on als Moschusriechstoff. Beschrieben werden zudem Riech- oder Aromastoffmischungen umfassend (**Z**)-7-Cyclohexadecen-1-on und einen oder mehrere weitere Riech- oder Aromastoffe.

Die Offenlegungsschrift EP 2 662 098 A1 betrifft die Verwendung bestimmter Verbindungen zum Maskieren oder Vermindern des bzw. eines oder mehrerer unangenehmer Geruchseindrücke eines oder mehrerer unangenehm riechender Stoffe und/oder zum Verstärken des bzw. eines oder mehrerer angenehmer Geruchseindrücke eines oder mehrerer angenehm riechender Stoffe sowie neue Riechstoffmischungen und parfümierte Produkte enthaltend solche Verbindungen.

Das US-Patent 4,568,470 A beschreibt die Verwendung von (**E**)-, (**Z**)- oder (**E**,**Z**)-Oxacyclohexadec-11-en-2-on zur Verstärkung des Aromas oder Geschmacks von Konsumgütern wie z.B. Parfümkompositionen, Nahrungsmitteln, Kaugummis, Zahnpasten und medizinischen Produkten.

Es war daher primäre Aufgabe der vorliegenden Erfindung, alternative oder vorzugsweise verbesserte Substanzen (Stoffe oder Stoffmischungen) zur Veränderung bzw. Beeinflussung geruchlicher Aspekte bereitzustellen, insbesondere um in Kombination mit Aldehyden und Alkoholen positive Effekte anzugeben.

Diese Substanzen sollten dabei vorzugsweise eine, mehrere oder vorzugsweise sämtliche der folgenden Anforderungen erfüllen:
- leichte Zugänglichkeit,
- hohe Wirksamkeit in geringer Konzentration, vorzugsweise bei in geringen Konzentrationen nicht oder kaum wahrnehmbarem Eigengeruch,
- weitgehende oder vollständige Farblosigkeit,
- hohe Stabilität in diversen Mischungen bzw. Zubereitungen, wobei insbesondere keine Verfärbung und/oder Separation und/oder Trübung auftreten soll,
- inertes Verhalten,
- keine toxische und/oder allergene Wirkung gegenüber dem Menschen.

Des Weiteren sollten mit der vorliegenden Erfindung insbesondere neue, vorteilhafte Riechstoffmischungen, v.a. Parfümöle, angegeben werden, welche solche Substanzen enthalten. Solche Riechstoffmischungen sollten vorzugsweise dafür geeignet sein, bestimmte Produkte zu beduften beziehungsweise zu parfümieren.

Weiterhin sollten demensprechend parfümierte Produkte sowie Verfahren zur Herstellung solcher Produkte angegeben werden.

Weitere Aufgabenstellungen, die der vorliegenden Erfindung zugrundeliegen, ergeben sich aus den nachfolgenden Ausführungen und den beigefügten Patentansprüchen.

Die primäre Aufgabe der vorliegenden Erfindung wird gelöst durch eine Mischung, enthaltend oder bestehend aus trans- und cis-konfiguriertem Oxacyclohexadecen-2-on (Globalide, CAS No. 34902-57-3, 111879-80-2), wobei das Gewichtsverhältnis von der Verbindung (E) zu der Verbindung (Z) im Bereich von 10 : 90 bis 60 : 40 liegt,
oder
aus trans- und cis-konfiguriertem Oxacycloheptadec-10-en-2-on (Ambrettolide, CAS No. 28645-51-4), wobei das Gewichtsverhältnis von der Verbindung (E) zu der Verbindung (Z) im Bereich von 10 : 90 bis 60 : 40 liegt.

Überraschenderweise sind die neuen Mischungen (wie hierin beschrieben) sehr gut geeignet, um positive Einflüsse auf (andere) Riechstoffe zu bewirken, insbesondere auf solche Riechstoffe wie hierin beschrieben, v.a. Aldehyde und Alkohole.

Die erfindungsgemäß zu verwendenden Verbindungen bzw. die hierin beschriebenen Mischungen sind weiterhin vorteilhafterweise leicht zugänglich bzw. herstellbar. Bevorzugte Verfahren zur Herstellung einer erfindungsgemäßen Mischung sind weiter unten beschrieben.

Weiterhin weisen die erfindungsgemäß zu verwendenden Verbindungen bzw. die hierin beschriebenen erfindungsgemäßen Mischungen bereits in geringer Konzentration eine hohe Wirksamkeit auf, insbesondere bei Konzentrationen, bei denen die Verbindungen keinen oder zumindest lediglich einen kaum wahrnehmbarem Eigengeruch aufweisen, sie sind vorteilhafterweise weitgehend oder vollständig farblos, besitzen eine hohe Stabilität in unterschiedlichen Mischungen bzw. Zubereitungen und weisen keine toxische und/oder allergene Wirkung gegenüber dem Menschen auf.

Die hierin beschriebenen erfindungsgemäßen Mischungen haben zudem den Vorteil, dass sie bei ihrer Verwendung, insbesondere einer erfindungsgemäßen Verwendung, mit unterschiedlichen Riechstoffen und Parfümölen bzw. üblichen Bestandteilen eines Parfümöls kombiniert werden können, um Produkte mit einer beliebigen Duftrichtung zu parfümieren. Folglich lässt sich durch die vorliegende Erfindung den Verbrauchern gegenüber eine breite Auswahl an Dufttypen anbieten. Erfindungsgemäße Riechstoffmischungen und parfümierte Produkte werden weiter unten beschrieben.

In diesem Zusammenhang sei exemplarisch auf die folgenden Ergebnisse zur Untersuchung der olfaktorischen Eigenschaften erfindungsgemäßer Mischungen (und Vergleichsmischungen) verwiesen:
Die olfaktorischen Eigenschaften ausgewählter Isomerengemische von Cyclohexadec-8-en-1-on können wie folgt beschrieben werden:
- 67 % trans-Cyclohexadec-8-en-1-on : 31 % cis-Cyclohexadec-8-en-1-on (nicht erfindungsgemäß):
   moschusartig, erogen, kosmetisch, balsamisches Geruchsbild und florale Aspekte
- 60 % trans-Cyclohexadec-8-en-1-on : 40 % cis-Cyclohexadec-8-en-1-on (nicht erfindungsgemäß):
   moschusartig, erogen, balsamisch, an frische Waldluft erinnernd
- 40 % trans-Cyclohexadec-8-en-1-on : 60 % cis-Cyclohexadec-8-en-1-on (nicht erfindungsgemäß):
   moschusartig, erogen, transparent, an kühle Seeluft erinnernd
- 20 % trans-Cyclohexadec-8-en-1-on : 80 % cis-Cyclohexadec-8-en-1-on (nicht erfindungsgemäß):
   moschusartig, erogen, warm, an trockenes Holz erinnernd.

Die olfaktorischen Eigenschaften ausgewählter Isomerengemische von Oxacycloheptadec-10-en-2-on können wie folgt beschrieben werden:
- 99 % trans-Oxacycloheptadec-10-en-2-on : 1 % cis-Oxacycloheptadec-10-en-2-on (Vergleichsmischung):
   moschusartig, erogen, mild ambra-ähnlich, mit floralen Aspekten und geringen fruchtigen Nuancen
- 65 % trans-Oxacycloheptadec-10-en-2-on : 30 % cis-Oxacycloheptadec-10-en-2-on (nicht erfindungsgemäß):
   moschusartig, erogen, stärker ambra-ähnlich mit mehr fettigen, animalischen Nuancen. Die floralen und fruchtigen Schattierungen treten olfaktiv in den Hintergrund.

Die olfaktorischen Eigenschaften ausgewählter Isomerengemische von Oxacyclohexadecen-2-on können wie folgt beschrieben werden:
- 72 % trans-Oxacyclohexadecen-2-on 22 % cis-Oxacyclohexadecen-2-on (Vergleichsmischung):
   moschusartig, erogen, fettig-ambraähnlich mit einer trocken-holzigen und dezentfruchtigen Ausrichtung
- 65 % trans-Oxacyclohexadecen-2-on : 31 % cis-Oxacyclohexadecen-2-on (nicht erfindungsgemäß):
   moschusartig, erogen, intensiver talgig-ambraähnlich, mehr an Haut, besonders an gereinigtes Wollfett erinnernd.

Die olfaktorischen Eigenschaften ausgewählter Isomerengemische von Cyclohexadec-5-en-1-on können wie folgt beschrieben werden:
- 37 % trans-Cyclohexadec-5-en-1-on : 62 % cis-Cyclohexadec-5-en-1-on (nicht erfindungsgemäß):
   moschusartig, erogen, mild-ambraähnlich moschusartig, erogen, mild-animalisch mit einem sanft-kosmetischen und transparent-floralem Geruchsbild
- 21 % trans-Cyclohexadec-5-en-1-on : 78 % cis-Cyclohexadec-5-en-1-on (nicht erfindungsgemäß):
   moschusartig, erogen, schwächer animalisch mit einer balsamischen, warmen, an Baum- und Strauchharze erinnernden Duftrichtung.

Vorzugsweise handelt es sich bei einer erfindungsgemäßen Mischung um eine Riechstoffmischung, insbesondere ein Parfümöl. Eine solche Riechstoffmischung enthält vorzugsweise einen oder mehrere zusätzliche Riechstoffe. Besonders bevorzugt ist bzw. sind der zusätzliche bzw. einer, mehrere oder sämtliche der zusätzlichen Riechstoffe ausgewählt aus der Gruppe bestehend aus Alkoholen, Aldehyden, Ketonen, Ethern, Estern und Carboxylaten, vorzugsweise Alkoholen und Aldehyden, insbesondere solchen mit einer Molmasse im Bereich von 150 bis 285 g/mol, vorzugsweise von 210 g/mol oder weniger. Im Zusammenhang mit der vorliegenden Erfindung gilt generell, dass die in einer erfindungsgemäßen Riechstoffmischung gegebenenfalls zusätzlich enthaltenen Riechstoffe eine Molmasse im Bereich von 150 bis 285 g/mol, vorzugsweise von 210 g/mol oder weniger, besitzen. Besonders bevorzugt zu verwendende Riechstoffe werden weiter unten beschrieben.

Im Folgenden werden - beispielhaft anhand exemplarisch untersuchter Isomerengemische von Cyclohexadec-8-en-1-on (wie oben beschrieben) - positive Einflüsse der hierin beschriebenen Mischungen auf ausgewählte, sogenannte "Messriechstoffe" (molekulare Einzelriechstoffe als Vertreter der chemisch-funktionellen Gruppe der Aldehyde und Alkohole) beschrieben:

### Aldehyde:

Dodecanal ist ein aliphatischer Aldehyd mit einem fettig, wachsigen Geruch, der oft auch als typischer "Frischwäscheduft" bezeichnet wird.

Bei einem Zusatz von 10 % (bezogen auf das Gesamtgewicht an Dodecanal) eines Isomerengemisches aus Cyclohexadec-8-en-1-on (hier: 40 % trans-Cyclohexadec-8-en-1-on 60 % cis-Cyclohexadec-8-en-1-on) zu diesem Einzelriechstoff ist überraschenderweise ein sehr positiver Geruch nach Citrusfrüchten, speziell nach Mandarine feststellbar.

2-Methyl Undecanal, auch als iso-Dodencal oder "M.N.A" bekannt, besitzt ein sehr kräftiges Geruchsbild, das mit "trocken", "ambraähnlich" und "krautig" beschrieben wird.

Ein Zusatz von 10% (bezogen auf das Gesamtgewicht an 2-Methyl Undecanal) eines Isomerengemisches aus Cyclohexadec-8-en-1-on (hier: 60 % trans-Cyclohexadec-8-en-1-on : 40 % cis-Cyclohexadec-8-en-1-on) überzeugt mit diffusivem, an Orangenschalen erinnernden Duftcharakter.

Hexylzimtaldehyd, alpha, ist eine iso-cyclische Aldehydform mit einem milden, leicht herbalen und schwach floralem Geruchsbild und erfährt durch den Zusatz von 10 % (bezogen auf das Gesamtgewicht an Hexylzimtaldehyd) eines Isomerengemisches aus Cyclohexadec-8-en-1-on (hier: 40 % trans-Cyclohexadec-8-en-1-on : 60 % cis-Cyclohexadec-8-en-1-on) eine höherwertige, fein-blumige Geruchsentwicklung nach frischen Blüten des Jasminstrauchs.

### Alkohole:

Dimethyloctenol, allgemein bekannt als "Citronellol", zeigt in seiner racemischen Form einen Geruch nach Rosenblüten und Maiglöckchen.

Ergänzt man diesen molekularen Einzelriechstoff mit einem Zentel seines Anteils durch ein Isomerengemisch aus Cyclohexadec-8-en-1-on (hier: 40 % trans-Cyclohexadec-8-en-1-on : 60 % cis-Cyclohexadec-8-en-1-on), so erhält man wider Erwarten den klaren, frischen, leicht minzigen Geruchseindruck der Laevo-Form des Moleküls, das deutlich nach den frischen Blättern der Duftgeranienstaude riecht.

Dimethyloctadienol, industriell bekannt als "Linalool", mit dem authentischen, blumigholzigen Geruch mit leichtem Citrus-Einfluss, gewinnt durch den Zusatz von 10 % (bezogen auf das Gesamtgewicht an Dimethyloctadienol) eines Isomerengemisches aus Cyclohexadec-8-en-1-on (hier: 60 % trans-Cyclohexadec-8-en-1-on : 40 % cis-Cyclohexadec-8-en-1-on) den elegant-fruchtigen Dufteindruck der Freesienblüte.

Die vorstehend beschriebenen "Messriechstoffe" sind im Rahmen der vorliegenden Erfindung besonders bevorzugt in Kombination mit Oxacyclohexadecen-2-on oder Oxacycloheptadec-10-en-2-on in erfindungsgemäßen Riechstoffmischungen einzusetzende Riechstoffe.

Im Zusammenhang mit den hierin beschriebenen erfindungsgemäßen Riechstoffmischungen gilt, dass das Verhältnis der Gesamtmasse an nicht Oxacyclohexadecen-2-on und Oxacycloheptadec-10-en-2-on entsprechenden Riechstoffen zur Gesamtmasse an Oxacyclohexadecen-2-on und Oxacycloheptadec-10-en-2-on vorzugsweise größer oder gleich 80 : 20 ist, bevorzugt größer oder gleich 90 : 10, besonders bevorzugt größer oder gleich 95 : 5.

Erfindungsgemäße Riechstoffmischungen sind üblicherweise bei 25°C und 1013hPa flüssig und in der Regel homogene Lösungen.

Riechstoffmischungen, insbesondere Parfümöle, umfassen häufig synthetische oder natürliche (vorzugsweise) geschmacks- und geruchsneutrale Trägeröle, welche die Duftbeziehungsweise Riechstoffe (als künstliche oder natürliche Stoffe) in hochkonzentrierter Form (sowie gegebenenfalls parfümistische Lösemittel und/oder Hilfsstoffe) enthalten. Entsprechendes gilt für die hierin beschriebenen erfindungsgemäßen Riechstoffmischungen.

Parfümöle (als bevorzugte Ausgestaltung von (erfindungsgemäßen) Riechstoffmischungen) dienen häufig Duftanwendungen. Mit Parfümölen werden beispielsweise Parfüme hergestellt, indem man sie in (z.B. alkoholische) Lösungen gibt, die beim Verdampfen die Duft- beziehungsweise Riechstoffe "mitreißen" und so dem Riechorgan des Anwenders, d.h. des Menschen, den Sinneseindruck eines bestimmten Geruchs vermitteln. Solche Mischungen können beispielsweise ein Parfüm, Eau de Parfüm oder Eau de Toilette sein. Ferner dienen Parfümöle der Erzeugung eines bestimmten Duftes in Wohnräumen, wie beispielsweise bei der Anwendung in Duftlampen, Zerstäubern oder Diffuser. Darüber hinaus können Parfümöle aber auch in unzähligen weiteren Artikeln bzw. Zubereitungen eingesetzt werden, beispielsweise von Schuhcremes bis Haarshampoos, Damenbinden bis WC-Reinigern, Gesichtscremes bis Waschpulver und Katzensteinen.

Beispiele für Riechstoffe, die grundsätzlich vorteilhaft als Bestandteil einer erfindungsgemäßen Riechstoffmischung, insbesondere eines erfindungsgemäßen Parfümöls, verwendet werden können, finden sich z.B. in S. Arctander, Perfume and Flavor Chemicals, Vol. I und II, Montclair, N. J., 1969, Selbstverlag oder H. Surburg, J. Panten, Common Fragrance and Flavor Materials, 5th Ed., Wiley-VCH, Weinheim 2006.

Bevorzugte Etherische Öle, Concretes, Absolues, Resine, Resinoide, Balsame und/oder Tinkturen, die Bestandteil einer erfindungsgemäßen Riechstoffmischung, insbesondere eines erfindungsgemäßen Parfümöls, sein können, sind bevorzugt auszuwählen aus der Gruppe bestehend aus:
Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-ÖI; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teebaum-Öl; Terpentinöl (engl.: turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl.

Bevorzugte Einzelriechstoffe, die vorzugsweise als Bestandteil einer erfindungsgemäßen Riechstoffmischung, insbesondere eines erfindungsgemäßen Parfümöls, verwendet werden, sind ausgewählt aus der Gruppe
der Kohlenwasserstoffe, dabei bevorzugt 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole, dabei bevorzugt Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale, dabei bevorzugt Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime, dabei bevorzugt 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen, dabei bevorzugt 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile, dabei bevorzugt 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren, dabei bevorzugt (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole, dabei bevorzugt Citronellol; Geraniol; Nerol; Linalool; Lavandulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone, dabei bevorzugt Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole, dabei bevorzugt Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; Menthylformiat; Menthylpropionat; Menthylbutyrat; Menthylisobutyrat; Menthylisovalerianat; Menthylhexanoat; Menthylcrotonat; Menthyltiglinat;
der cyclischen Terpenaldehyde und -ketone, dabei bevorzugt Menthon; Isomenthon; 8-Mercaptomenthan-3-on; Carvon; Campher; Fenchon; alpha-Ionon; beta-Ionon; beta-n-Methylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole, dabei bevorzugt 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole, dabei bevorzugt alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether, dabei bevorzugt Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone, dabei bevorzugt 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde, dabei bevorzugt 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone, dabei bevorzugt 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole, dabei bevorzugt 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole, vorzugsweise 1-Cyclohexylethylcrotonat;
der Ester cycloaliphatischer Carbonsäuren, dabei bevorzugt Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole, dabei bevorzugt Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentan-1-ol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren, dabei bevorzugt Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether, dabei bevorzugt 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde, dabei bevorzugt Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone, dabei bevorzugt Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester, dabei bevorzugt Benzoesäure; Phenylessigsäure; Methylbenzoat; Ethylbenzoat; Hexylbenzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen, dabei bevorzugt 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiffsche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester, dabei bevorzugt Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen, dabei bevorzugt 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone, dabei bevorzugt 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,13-tridecandioat; Cumarin; 2,3-Dihydrocumarin; Octahydrocumarin.

Besonders bevorzugt ist eine erfindungsgemäße Riechstoffmischung, wobei die Menge an erfindungsgemäßer Mischung bzw. die Menge an Verbindung (**E**) und Verbindung (**Z**) (wie hierin beschrieben) ausreicht,
(a) um den bzw. einen oder mehrere unangenehme Geruchseindrücke eines anderen Riechstoffs in der Riechstoffmischung zu maskieren oder zu vermindern,
   und/oder
(b) um den bzw. einen oder mehrere angenehme Geruchseindrücke eines anderen Riechstoffs in der Riechstoffmischung zu verstärken.

Besonders bevorzugt ist es auch, wenn die Gesamtmenge an Oxacyclohexadecen-2-on und Oxacycloheptadec-10-en-2-on bezogen auf das Gesamtgewicht der Riechstoffmischung 1 Gew.-% oder weniger beträgt, vorzugsweise 0,1 Gew.-% oder weniger, besonders bevorzugt 0,001 Gew.-% oder weniger.

Ein weitererAspekt der vorliegenden Erfindung betrifft ein parfümiertes Produkt, enthaltend eine erfindungsgemäße Mischung wie hierin beschrieben, vorzugsweise eine erfindungsgemäße Riechstoffmischung, insbesondere ein Parfümöl, in einer sensorisch wirksamen Menge, wobei der Anteil der Mischung bzw. der Riechstoffmischung oder an Oxacyclohexadecen-2-on und Oxacycloheptadec-10-en-2-on bezogen auf das Gesamtgewicht des Produkts vorzugsweise im Bereich von 0,01, bevorzugt 0,1, bis 20, bevorzugt 10 Gew.-%, bevorzugt im Bereich von 0,1 bis 5 Gew.-%, besonders bevorzugt im Bereich von 0,25 bis 3 Gew.-%, liegt.

Für einzusetzende bzw. enthaltene Verbindungen Oxacyclohexadecen-2-on oder Oxacycloheptadec-10-en-2-on sowie gegebenenfalls die weiteren Bestandteile einer enthaltenen Riechstoffmischung gilt das oben Gesagte entsprechend.

Klarstellend ist zu erwähnen, dass erfindungsgemäße (parfümierte) Produkte im Rahmen des vorliegenden Textes als absichtlich herbeigeführte bzw. hergestellte Produkte zu verstehen sind, jedoch nicht als natürlich vorkommende Stoffmischungen, wie sie zum Beispiel durch Extraktion von pflanzlichen Ausgangsmaterialien gewonnen werden können.

Bevorzugte Produkte sind beispielsweise Parfüm-Extraits, Eau de Parfums, Eau de Toilettes, Rasierwässer, Eau de Colognes, Pre-shave-Produkte, Splash-Colognes und parfümierte Erfrischungstücher sowie parfümierte oder zu parfümierende saure, alkalische und neutrale Reinigungsmitteln, wie z.B. Fußbodenreiniger, Fensterglasreiniger, Geschirrspülmittel, Bad- und Sanitärreiniger, Scheuermilch, feste und flüssige WC-Reiniger, WC-Sticks, WC-Steine (flüssig oder fest), pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseife, Waschtabletten, Desinfektionsmittel, Oberflächendesinfektionsmittel sowie Luftverbesserer in flüssiger, gelartiger oder auf einem festen Träger aufgebrachter Form, besonders zur Desodorierung von Abluft aus Klimaanlagen und industriellen Prozessen, sowie Luftverbesserer in Form von Aerosol- oder Pumpsprays, Wachse und Polituren wie Möbelpolituren, Fußbodenwachse, Schuhcremes, stärkende, imprägnierende oder desodorierende Textilbehandlungsmittel, Windeln, Monatsbinden, Slip-Einlagen, Pflaster, sowie Körperpflegemittel wie z.B. feste und flüssige Seifen, Duschgele, Shampoos, Rasierseife, Rasierschäume, Badeöle, Feuchtreinigungstücher, kosmetische Emulsionen vom Öl-in-Wasser-, vom Wasser-in-ÖI- und vom Wasser-in-ÖI-in-Wasser-Typ wie z.B. Hautcremes und -lotionen, Gesichtscremes und -lotionen, Sonnenschutzcremes und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukte wie z.B. Haarsprays, Haargele, festigende Haarlotionen, Haarspülungen, permanente und semipermanente Haarfärbemitteln, Haarverformungsmittel wie Kaltwellen- und Haarglättungsmittel, Haarwässer, Haarcremes und -lotionen, Deodorantien und Antiperspirantien wie z.B. Achsel-sprays, Roll-ons, Deosticks, Deocremes, Produkte der dekorativen Kosmetik wie z.B. Lidschatten, Make-ups, Lippenstifte, Mascara sowie Kerzen, Lampenöle, Räucherstäbchen, Tierstreu, Katzenstreu, Insektizide, Repellentien, flüssige und gasförmige Treibstoffe, Heizöle und Heizgase.

Besonders bevorzugt ist ein erfindungsgemäßes Produkt ausgewählt aus der Gruppe bestehend aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und - lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

Einem erfindungsgemäßen Produkt kann auch ein durch eine Verminderung eines unangenehmen Geruchs (insbesondere wie oben beschrieben) oder durch eine Verstärkung eines angenehmen geruchlichen Aspekts (insbesondere wie hierin beschrieben) geruchlich zu verbesserndes Produkt zugrundeliegen.

Gemäß einer bevorzugten Ausgestaltung sind die erfindungsgemäß einzusetzenden Verbindungen Oxacyclohexadecen-2-on oder Oxacycloheptadec-10-en-2-on beziehungsweise entsprechende Mischungen davon oder Riechstoffmischungen (wie hierin beschrieben) an einem Trägerstoff adsorbiert, der sowohl für eine feine Verteilung der Verbindungen im Produkt als auch für eine kontrollierte Freisetzung bei der Anwendung sorgt. Derartige Träger können poröse anorganische Materialien wie Kieselgele, Zeolithe, Gipse, Tone, Tongranulate, Gasbeton usw. oder organische Materialien wie Hölzer und Cellulose-basierende Stoffe sein.

Die erfindungsgemäß einzusetzenden Verbindungen Oxacyclohexadecen-2-on oder Oxacycloheptadec-10-en-2-on beziehungsweise entsprechende Mischungen davon oder Riechstoffmischungen (wie hierin beschrieben) können auch mikroverkapselt, sprühgetrocknet, als Einschluss-Komplexe oder als Extrusions-Produkte vorliegen und in dieser Form einem Produkt hinzugefügt werden.

Gegebenenfalls können die Eigenschaften solcher derart modifizierten erfindungsgemäß zu verwendenden Verbindungen Oxacyclohexadecen-2-on oder Oxacycloheptadec-10-en-2-on beziehungsweise entsprechender Mischungen davon oder Riechstoffmischungen (wie hierin beschrieben) durch sogenanntes "Coaten" mit geeigneten Materialien im Hinblick auf eine gezieltere Freisetzung weiter optimiert werden, wozu vorzugsweise wachsartige Kunststoffe wie z.B. Polyvinylalkohol verwendet werden.

Eine Mikroverkapselung der erfindungsgemäß einzusetzenden Verbindungen Oxacyclohexadecen-2-on oder Oxacycloheptadec-10-en-2-on beziehungsweise entsprechender Mischungen davon oder Riechstoffmischungen (wie hierin beschrieben) kann beispielsweise durch das sogenannte Koazervationsverfahren mit Hilfe von Kapselmaterialien, z.B. aus polyurethan-artigen Stoffen oder Weichgelatine, erfolgen. Sprühgetrocknetes Oxacyclohexadecen-2-on oder Oxacycloheptadec-10-en-2-on kann beispielsweise durch Sprühtrocknung einer erfindungsgemäß einzusetzende Substanz hergestellt werden, wobei als Trägerstoff modifizierte Stärken, Proteine, Dextrin und/oder pflanzliche Gummen verwendet werden können. Einschluss-Komplexe können z.B. durch Eintragen von Dispersionen, welche erfindungsgemäß einzusetzende Verbindungen Oxacyclohexadecen-2-on oder Oxacycloheptadec-10-en-2-on oder entsprechende Mischungen davon sind beziehungsweise solche umfassen, und Cyclodextrinen oder Harnstoffderivaten in ein geeignetes Lösungsmittel, z.B. Wasser, hergestellt werden. Extrusions-Produkte können durch Verschmelzen der erfindungsgemäß einzusetzenden Verbindung(en) Oxacyclohexadecen-2-on oder Oxacycloheptadec-10-en-2-on beziehungsweise entsprechender Mischungen mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäß einzusetzenden Verbindungen Oxacyclohexadecen-2-on oder Oxacycloheptadec-10-en-2-on beziehungsweise entsprechende Mischungen davon oder Riechstoffmischungen (wie hierin beschrieben) können in vielen Zubereitungen bzw. Produkten eingesetzt werden, wobei sie vorzugsweise mit einem oder mehreren der folgenden Hilfs- oder Wirkstoffe kombiniert werden:
Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, (Textil-) stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UVabsorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Gemäß einer Ausgestaltung der vorliegenden Erfindung enthält ein bevorzugtes erfindungsgemäßes Produkt, insbesondere ein Deodorant oder dergleichen, (je nach gewünschter Wirkweise) zudem einen oder mehrere der folgenden Wirkstoffe:
(1) Antimikrobiell wirksame Substanzen, die die Entwicklung der für Schweißgeruch verantwortlichen Mikroorganismen hemmen; beispielsweise Triclosan^{®} (5-Chlor-2-(2,4-dichlorphenoxy)phenol), Triclocarban, Chlorhexidin, Chlorhexidinhydrochlorid, Chlorhexidindiacetat, Chlorhexidindigluconat, 2-Phenoxyethanol, Farnesol, Glycerinester und -ether wie Glycerinmonolaurat, Glycerinmonocaprinat, Hexoxyglycerin, Octoxyglycerin (= Ethylhexylglycerin, 3-(2-Ethylhexyloxy-1,2-Propandiol) oder Sensiva^{®} SC 50 (von Schülke & Mayr), aliphatische 1,2-Diole wie z.B. 1,2-Decandiol (EP 1 269 983), araliphatische Alkohole wie beispielsweise beschrieben in EP 799 174, vorzugsweise 4-Methyl-4-phenyl-2-pentanol (Vetikol; WO 03/024907) oder 2-Methyl-4-phenyl-2-butanol (1,1-Dimethyl-3-phenylpropanol, alpha,alpha-Dimethylphenethylcarbinol), I-Menthylmethylether wie beschrieben in WO 02/41861, 2-Benzylheptan-1-ol (Jasmol; 2-n-Pentyl-3-phenylpropan-1-ol), 2,2-Dimethyl-3-phenylpropanol (Muguetalkohol; vgl. US 4,091,090), antimikrobiell wirksame sekundäre Alkohol, wie beispielsweise beschrieben in WO 2005/004601, insbesondere 3-Methyl-6-phenyl-2-hexanol, 4-(2,4-Dimethylphenyl)-2-butanol, 6-(4-Isopropylphenyl)-3-methyl-2-hexanol, 4-(2,4,5-Trimethylphenyl)-2-butanol, 3,3-Dimethyl-4-phenyl-2-butanol, 3-Methyl-4-(2-methylphenyl)-2-butanol, 6-(3,4-Dimethylphenyl)-2-hexanol, aliphatische Carbonsäuren wie 2-Hexyloctansäure, 2-Hexyldecansäure, 2-Butyloctansäure oder 2-Butyldecansäure;
(2) enzyminhibierende Substanzen, die die Wirkung von Enzymen, die an der Bildung von Schweißgeruch beteiligt sind, unterbinden; beispielsweise Citronensäureester und metall-chelatisierende Substanzen wie EDTA (Ethylendiamintetraessigsäure), EGTA [Ethylenbis(oxyethylennitrilo)-tetraessigsäure] und DTPA (Diethylentriaminpentaessigsäure, Pentetic acid);
(3) geruchsabsorbierende Substanzen, die für den Schweißgeruch verantwortliche Stoffe absorbieren; beispielsweise Zinkrizinoleat, Cyclodextrine;
(4) Antiperspirantien, welche die Schweißsekretion hemmen und damit den für den Körpergeruch verantwortlichen Bakterien den Nährboden entziehen. Als Antiperspirantien verwendet man im allgemeinen vorzugsweise adstringierende Metallsalze, besonders anorganische und organische Metallsalze der Elemente Aluminium, Zink, Magnesium, Zinn und Zircon sowie deren Mischungen, wobei insbesonders Halogenide wie Aluminiumchlorid, basische Aluminiumhydroxychloride, Zirconyloxychloride und Zirconylhydroxychloride sowie deren Mischungen verwendet werden. Häufig werden diese Aluminium- und Zirconiumsalze und deren Mischungen auch in einer komplexierten Form verwendet, wobei als Komplexbildner vorzugsweise Propylenglykol, Polyethylenglykol oder Glycin verwendet werden.

Die vorliegende Erfindung betrifft auch ein Verfahren zum Herstellen eines parfümierten Produkts, insbesondere eines erfindungsgemäßen parfümierten Produkts wie hierin beschrieben, umfassend folgende Schritte:
i) Bereitstellen einer erfindungsgemäßen Mischung oder Riechstoffmischung in einem Gewichtsverhältnis wie hierin erfindungsgemäß definiert,
ii) Bereitstellen eines oder mehrerer weiterer Bestandteile des herzustellenden parfümierten Produkts (insbesondere solcher wie oben beschrieben), und
iii) Inkontaktbringen oder Mischen der in Schritt ii) bereitgestellten weiteren Bestandteile mit einer sensorisch wirksamen Menge der in Schritt i) bereitgestellten Bestandteile.

Wie sich aus den Ausführungen weiter oben ergibt eignen sich erfindungsgemäße Mischungen als Geruchsmodifizierer, vorzugsweise
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe,
insbesondere in Kombination mit (anderen) Riechstoffen wie weiter oben beschrieben.

Bevorzugt ist hierbei die Verwendung einer erfindungsgemäßen Mischung in einer Zusammensetzung, vorzugsweise einem Parfümöl, die bzw. das einen oder mehrere (weitere) angenehm und/oder unangenehm riechende Stoffe enthält, deren unangenehmer Geruchseindruck durch die erfindungsgemäße Mischung maskiert oder vermindert und/oder deren angenehmer Geruchseindruck durch die erfindungsgemäße Mischung verstärkt wird, wobei diese(r) angenehm und/oder unangenehm riechende Stoff bzw. einer, mehrere oder sämtliche dieser angenehm und/oder unangenehm riechende Stoffe ausgewählt ist bzw. sind aus der Gruppe bestehend aus Alkoholen, Aldehyden, Ketonen, Ethern, Estern und Carboxylaten, vorzugsweise Ketonen und Estern, und/oder eine Molmasse im Bereich von 150 bis 285 g/mol hat bzw. haben.

Hierin beschrieben wird in diesem Zusammenhang auch ein Verfahren
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe,
umfassend folgenden Schritt:
Vermischen des bzw. der (a) angenehm und/oder (b) unangenehm riechenden Stoffe mit einer erfindungsgemäßen Mischung oder Riechstoffmischung in einem Gewichtsverhältnis wie hierin erfindungsgemäß definiert, wobei die Menge an der erfindungsgemäßen Mischung ausreicht um (a) den bzw. die angenehmen Geruchseindrücke des bzw. der angenehm riechenden Stoffe zu verstärken und/oder (b) den bzw. die unangenehmen Geruchseindrücke des bzw. der unangenehm riechenden Stoffe zu vermindern oder zu maskieren.

Wie einleitend erwähnt wird im Rahmen der vorliegenden Erfindung auch ein Verfahren zur Herstellung einer Mischung enthaltend oder bestehend aus trans- und cis-konfiguriertem Oxacyclohexadecen-2-on, wobei das Gewichtsverhältnis von der Verbindung (**E**) zu der Verbindung (**Z**) im Bereich von 10 : 90 bis 60 : 40 liegt, oder aus trans- und cis-konfiguriertem Oxacycloheptadec-10-en-2-on, wobei das Gewichtsverhältnis von der Verbindung (**E**) zu der Verbindung (**Z**) im Bereich von 10 : 90 bis 60 : 40 liegt, bereitgestellt. Ein solches Verfahren umfasst wenigstens folgende Schritte:
(i) Bereitstellen einer Ausgangsmischung enthaltend oder bestehend aus trans- und cis-konfiguriertem Oxacyclohexadecen-2-on, wobei das Gewichtsverhältnis von der Verbindung (**E**) zu der Verbindung (**Z**) in der Ausgangsmischung größer als 60 : 40 ist,
   oder
   aus trans- und cis-konfiguriertem Oxacycloheptadec-10-en-2-on, wobei das Gewichtsverhältnis von der Verbindung (**E**) zu der Verbindung (**Z**) in der Ausgangsmischung größer als 60 : 40 ist,
(ii) Durchführen einer säurekatalysierten Isomerisierung, vorzugsweise unter Verwendung von Salpetersäure, der in der Ausgangsmischung enthaltenen Verbindung (**E**), so dass eine Mischung erhalten wird, worin das Gewichtsverhältnis von der Verbindung (**E**) zu der Verbindung (**Z**) in der Mischung im Bereich von 10 : 90 bis 60 : 40 liegt.

Bevorzugte Ausgestaltungen dieses Verfahrens bzw. bevorzugt einzusetzende Verbindungen ergeben sich aus der vorangehenden Beschreibung. Besonders bevorzugt handelt es sich bei einer hierin beschriebenen erfindungsgemäßen Mischung um eine Mischung, die unter Anwendung eines erfindungsgemäßen Verfahrens hergestellt bzw. herstellbar ist.

Eine beispielhafte Verfahrensführung eines nicht-erfindungsgemäßen Verfahrens ist in Beispiel 3 beschrieben.

Anreicherungen von Z-Isomer lassen sich auch durch eine fraktionierte Destillation erzielen (siehe auch hierzu den Beispielteil unten).

Soweit nicht anders angegeben beziehen sich im Folgenden alle Angaben auf das Gewicht.

### Beispiele:

**Vergleichsbeispiel 1: Parfümöl P1 Aldehyde**

| **Bestandteil** | **Gew.-Anteil** |
|---|---|
| ALDEHYD C 11 10% in DPG | 16 |
| ALDEHYD C 11 UNDECYLEN 10% in DPG | 18 |
| ALDEHYD C 12 LAURIN 10% in DGP | 14 |
| ALDEHYD C 12 MNA 10% in DGP | 12 |
| HEXENAL TRANS-2 10% in DPG | 1,5 |
| HEXENYLACETAT CIS-3 | 4 |
| VERTOCITRAL | 7,5 |
| MAGNOLAN | 130 |
| MINTONAT | 35 |
| DIHYDROMYRCENOL | 70 |
| ORANGENOEL | 30 |
| NEROLIONE 10% in DPG | 1 |
| HEXYLACETAT | 30 |
| JASMAPRUNAT | 18 |
| ALDEHYD C14 SOG | 50 |
| FRUITATE | 1,8 |
| ETHYLMETHYLBUTYRAT-2 | 8 |
| MANZANATE | 1,2 |
| ALLYLCYCLOHEXYLPROPIONAT | 7 |
| APRIFLOREN | 2 |
| DUPICAL 10% in DPG | 3,5 |
| ETHYLLINALOOL | 57 |
| DIMETHYLBENZYLCARBINYLBUTYRAT | 6,5 |
| ROSE ABS. | 30 |
| ROSAFEN | 20 |
| DAMASCENON | 1,2 |
| DAMASCON ALPHA | 1,8 |
| BENZYLACETAT | 28 |
| HEDION | 60 |
| HEXYLZIMTALDEHYD ALPHA | 140 |
| PARMANYL | 3,5 |
| MYSOREACETAT | 35 |
| ISORALDEIN 70 | 28 |
| ISOEUGENOLMETHYLETHER | 3,5 |
| HELIOTROPIN | 3 |
| AGRUMEX LC | 70 |
| AMBROCENIDE 10% in DPG | 0,6 |
| AMBROXIDE | 1,4 |
| | 950 |

Die Komposition ist zu beschreiben als aldehydige, frisch-luftige mit blattgrünen, pfirsichartigen und ambraählichen Dufteindrücken.

Ein Zusatz von 10% (bezogen auf das Gesamtgewicht der in der beurteilten Riechstoffmischung enthaltenen Riechstoffe der Gruppe der Aldehyde) eines Isomerengemisches aus Cyclohexadec-8-en-1-on (hier: 60 % trans-Cyclohexadec-8-en-1-on : 40 % cis-Cyclohexadec-8-en-1-on) resultiert in einer deutlichen, so nicht erwarteten Zunahme der grünen und fruchtigen Dufteindrücke, die an grüne Äpfel erinnern.

**Vergleichsbeispiel 2: Parfümöl P2 Alkohole**

| **Bestandteil** | **Gew.-Anteil** |
|---|---|
| ALDEHYD C 12MNA | 5 |
| LINOLAL | 10 |
| VERTOCITRAL | 5 |
| GALBANUM RESIN SYNTH. | 5 |
| MAGNOLAN | 25 |
| MINTONAT | 30 |
| DIHYDROMYRCENOL | 50 |
| ORANGENOEL | 90 |
| PETITGRAINOEL PARAG. | 10 |
| LAVANDIN GROSSO RCO | 30 |
| HERBOXAN | 30 |
| FREESIOL | 30 |
| LINALOOL | 70 |
| GERANIUM RCO | 30 |
| PHENYLETHYLACETAT | 10 |
| PHENYLETHYLALKOHOL | 70 |
| CITRONELLOL | 20 |
| GERANIOL | 20 |
| ROSAFEN | 60 |
| BENZYLACETAT | 50 |
| AMYLZIMTALDEHYD ALPHA | 60 |
| YLANG MC TYPE BASE | 20 |
| AMYLSALICYLAT | 50 |
| PARMANYL | 5 |
| ISORALDEIN 70 | 30 |
| EUGENOL | 10 |
| ANISALDEHYD | 10 |
| HELIOTROPIN | 10 |
| CINNAMYLACETAT | 10 |
| CUMARIN | 10 |
| CYCLABUTE | 10 |
| HERBAFLORAT | 50 |
| PATCHOULIOEL | 10 |
| AMBROXIDE | 5 |
| CITRYLAL | 10 |
| | 950 |

Die Komposition ist zu beschreiben als ein floraler Akkord aus frischen Gartenblumen mit citrischen und an Gartenkräuter erinnernden Duftnoten.

Der Zusatz von 10 % (bezogen auf das Gesamtgewicht der in der beurteilten Riechstoffmischung enthaltenen Riechstoffe der Gruppe der Alkohole) eines Isomerengemisches aus Cyclohexadec-8-en-1-on (hier: 40 % trans-Cyclohexadec-8-en-1-on : 60 % cis-Cyclohexadec-8-en-1-on) lässt die Komposition sehr viel lebendiger, leichter blumig und weniger herbal erscheinen.

### Vergleichsbeispiel 3: Herstellung einer Mischung (Anreicherung von Z-Isomer durch Isomerisierung (z.B. unter Verwendung von Globalide, Ambrettolide oder Velvione))

45g Ambrettolide (Oxacycloheptadec-10-en-2-on) (hier: 99% (E) und 0,6% (Z)) werden in 225 ml Isopropanol gelöst und mit 3,0g Salpetersäure (65%ig) versetzt. Anschließend wird unter RT 8h gerührt.

Aufarbeitung: Der Ansatz wird mit Soda versetzt, am Rotationsverdampfer eingeengt und mittels KR (Kugelrohrdestillation) destilliert.

In entsprechender Weise kann ausgehend von einem Isomerengemisch von Globalide oder Velvione - anstelle von Ambrettolide - verfahren werden (für Details siehe unten angeführte Tabelle).

**Isomerisierungsergebnisse:**

| **Struktur** | **MG** | **GC-% Edukt** | **GC-% Isomerisierung** |
|---|---|---|---|
| **Globalide** | | | |
| 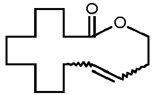 | 238 | 72% E | 65% E |
| | | 22% Z | 31% Z |
| **Ambrettolide** | | | |
| 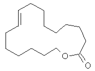 | 252 | 99,0% E | 67% E |
| | | 0,6% Z | 31% Z |
| **Velvione** | | | |
| 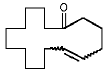 | 236 | 37% E | 21% E |
| | | 62% Z | 78% Z |

### Vergleichsbeispiel 4: Herstellung einer Mischung (Anreicherung von Z-Isomer durch Destillation (von z.B. Globanone))

### Apparatur:

4000ml DHK (Dreihalskolben), MGR (Magnetrührer), 1,5m Sulzerkolonne, Pilzheizhaube 2013,2g Globanone werden feindestilliert.

| | ST °C | KT °C | R:D | mbar | Ausbeute in g |
|---|---|---|---|---|---|
| Fraktion 1 bis | 187 | 112,2 | 80:2 | 3,8 | 32,6 |
| Fraktion 2 bis | 191 | 140,7 | 80:2 | 3,0 | 12,8 |
| Fraktion 3 bis | 193 | 141,5 | 80:2 | 3,0 | 31,4 |
| Fraktion 4 bis | 196 | 142,0 | 80:2 | 3,0 | 21,9 |
| Fraktion 5 bis | 197 | 142,3 | 80:2 | 3,0 | 80,3 |
| Fraktion 6 bis | 198 | 141,7 | 100:2 | 2,9 | 26,7 |
| Fraktion 7 bis | 198 | 141,9 | 100:2 | 3,0 | 68,2 |
| Fraktion 8 bis | 198 | 142,2 | 100:2 | 3,0 | 25,5 |
| Fraktion 9 bis | 199 | 141,5 | 80:2 | 3,0 | 86,4 |
| Fraktion 10 bis | 200 | 140,4 | 80:2 | 2,7 | 95,1 |
| Fraktion 11 bis | 198 | 140,6 | 80:2 | 2,7 | 84,3 |
| Fraktion 12 bis | 200 | 140,4 | 80:2 | 2,8 | 114,1 |

| Sumpfprobe 1 SP-1 | | | | | |
|---|---|---|---|---|---|
| Fraktion 13 bis | 199 | 141,6 | 80:2 | 2,9 | 249,4 |
| Fraktion 14 bis | 198 | 141,6 | 40:2 | 3,0 | 198,0 |
| Fraktion 15 bis | 198 | 142,5 | 40:2 | 3,1 | 193,2 |

| Sumpfprobe 2 SP-2 | | | | | |
|---|---|---|---|---|---|
| Fraktion 16 bis | 200 | 140,8 | 80:2 | 2,6 | 132,5 |
| Fraktion 17 bis | 203 | 139,7 | 80:2 | 2,6 | 83,6 |
| Fraktion 18 bis | 196 | 140,2 | 80:2 | 2,6 | 9,3 |
| Hold Up | - | - | - | - | 55,7 |
| Rückstand | - | - | - | - | 409 |

**Vom Rückstand werden 27,0g KR-destilliert**

| 27,0g Eins | T °C | R:D | mbar | Ausbeute in g |
|---|---|---|---|---|
| KRD-Fr. | 150 | 0:1 | 1,4 | 21,4 |
| KRD-Rü. | - | - | - | 5,8 verw. |

**"KRD-2"**

| 382g Eins | T °C | R:D | mbar | Ausbeute in g |
|---|---|---|---|---|
| KRD-Fr. | 153 | 0:1 | 0,9 | 303,4 |
| KRD-Rü. | - | - | - | 75,3 |

### KRD-2 wird nochmal destilliert!

Apparatur: 500ml DHK, MGR, 50cm FKK (Füllkörperkolonne) mit Wilsonspiralen, Pilzheizhaube

| *Einsatz KRD-2* | ST °C | KT °C | R:D | mbar | Ausbeute in g |
|---|---|---|---|---|---|
| RF | 176 | 109 | 1:0 | 0,5 | - |
| Fraktion 1 bis | 174 | 122 | 80:2 | 0,8 | 11,5 |
| Fraktion 2 bis | 176 | 125 | 80:2 | 0,9 | 13,6 |
| Fraktion 3 bis | 179 | 127 | 80:2 | 1,0 | 19,9 |
| Fraktion 4 bis | 180 | | 80:2 | 0,9 | 14,9 |
| Fraktion 5 bis | 177 | 130 | 80:2 | 1,1 | 24,0 |
| Fraktion 6 bis | 176 | 130 | 80:2 | 1,1 | 16,9 |
| Fraktion 7 bis | 175 | 129 | 80:2 | 1,1 | 13,2 |
| Fraktion 8 bis | 180 | 149 | 80:2 | 3,4 | 22,4 |

| Sumpfprobe SP-1 1 | | | | | |
|---|---|---|---|---|---|
| Fraktion 9 bis | 184 | 19 | 60:2 | 3,4 | 30,1 |
| Fraktion 10 bis | 182 | 130 | 60:2 | 1,0 | 36,2 |
| Fraktion 11 bis | 194 | 135 | 40:2 | 1,3 | 57,3 |
| Hold Up | - | - | - | - | 10,0 |
| Rückstand | - | - | - | - | 34,2 |

### Analysen:

### GC(FGC811)DB-1:

**Index Eins. Fr.1 Fr.2 Fr.3 Fr.4 Fr.5 Fr.6 Fr.7 Fr.8 Fr.9 Fr.10 %**

| *VL* | | *ca.28 3,0* | | 3,9 | 1,7 | *0,4* | *0,5* | *0,4* | *0,3* | *0,2* | *0,3* | *Vorlauf* |
|---|---|---|---|---|---|---|---|---|---|---|---|---|
| 1856 | | 4,2 | 4,2 | 3,4 | 3,1 | 2,0 | 1,6 | 1,2 | 1,1 | 0,9 | 0,8 | "7-E" |
| 1883 | 68,0 | 60,3 | 81,7 | 80,8 | 82,4 | 83,7 | 85,1 | 83,5 | 82,9 | 81,9 | 82,0 | trans |
| 1888 | 29,8 | 7,5 | 11,0 | 11,8 | 12,8 | 13,9 | 12,8 | 14,9 | 15,7 | 17,0 | 16,9 | cis |

**Index Eins. Fr.11 Fr.12 Fr.13 Fr.14 Fr.15 Fr.16 Fr.17 Fr.18 Rue HU %**

| *VL* | | *0,3* | *0,2* | 0,1 | 0,1 | - | - | - | *0,1* | - | *0,1* | *Vorlauf* |
|---|---|---|---|---|---|---|---|---|---|---|---|---|
| 1856 | | 0,7 | 0,5 | 0,5 | 0,3 | 0,2 | 0,2 | 0,2 | 0,2 | - | 0,2 | "7-E" |
| 1883 | 68,0 | 81,9 | 80,5 | 79,3 | 76,5 | 71,3 | 63,7 | 60,0 | 59,3 | 30,5 | 51,4 | trans |
| 1888 | 29,8 | 17,1 | 18,8 | 20,2 | 23,2 | 28,5 | 36,1 | 39,8 | 40,4 | 62,9 | 46,3 | cis |

**Index Eins. KRD KRD-2 %**

| *VL* | | - | - | *Vorlauf* |
|---|---|---|---|---|
| 1856 | | - | - | "7-E" |
| 1883 | 68,0 | 30,8 | 30,4 | trans |
| 1888 29,8 | | 62,6 | 63,2 | cis |
| *NL* | | 6,6 | *6,4* | *Nachlauf* |

### KRD-2-Destillation:

**Index KRD-2 Fr.1 Fr.2 Fr.3 Fr.4 Fr.5 Fr.6 Fr.7 Fr.8 SP-1 Fr.9 Fr.10 %**

| *VL* | - | *0,1* | - | *0,3* | - | - | - | - | - | - | - | - | *Vorlauf* |
|---|---|---|---|---|---|---|---|---|---|---|---|---|---|
| 1856 | - | - | 0,4 | 0,3 | - | 0,1 | 0,1 | 0,1 | 0,1 | - | 0,1 | 0,1 | "7-E" |
| 1883 | 30,4 | 34,2 | 35,2 | 40,2 | 35,1 | 34,9 | 35,7 | 33,9 | 32,9 | 26,9 | 32,0 | 32,7 | trans |
| 1888 | 63,2 | 63,4 | 62,4 | 54,8 | 63,1 | 63,1 | 62,0 | 63,8 | 64,3 | 62,5 | 65,5 | 62,2 | cis |
| *NL* | 6,4 | 2,3 | *2,0* | *4,4* | *1,8* | 1,9 | 2,2 | 2,2 | 2,7 | *10,6* | *2,4* | *5,0* | *Nachlauf* |

**Index KRD-2 Fr.11 Rue HU %**

| *VL* | - | - | - | - | *Vorlauf* |
|---|---|---|---|---|---|
| 1856 - | | 0,2 | 0,1 | 0,4 | "7-E" |
| 1883 | 30,4 | 28,6 | 15,4 | 24,7 | trans |
| 1888 63,2 | | 66,4 | 46,0 | 61,5 | cis |
| *NL* | 6,4 | 4,8 | *38,5* | *13,6* | *Nachlauf* |

## Patentansprüche

1. Mischung enthaltend oder bestehend aus
trans- und cis-konfiguriertem Oxacyclohexadecen-2-on, wobei das Gewichtsverhältnis von der Verbindung (**E**) zu der Verbindung (**Z**) im Bereich von 10 : 90 bis 60 : 40 liegt,
oder
aus trans- und cis-konfiguriertem Oxacycloheptadec-10-en-2-on, wobei das Gewichtsverhältnis von der Verbindung (**E**) zu der Verbindung (**Z**) im Bereich von 10 : 90 bis 60 : 40 liegt.

2. Mischung nach Anspruch 1, wobei die Mischung eine Riechstoffmischung ist, vorzugsweise ein Parfümöl, und zudem vorzugsweise einen oder mehrere zusätzliche Riechstoffe enthält.

3. Mischung nach Anspruch 2, wobei der zusätzliche bzw. einer, mehrere oder sämtliche der zusätzlichen Riechstoffe ausgewählt ist bzw. sind aus der Gruppe bestehend aus Alkoholen, Aldehyden, Ketonen, Ethern, Estern und Carboxylaten, vorzugsweise Aldehyden und Alkoholen.

4. Mischung nach Anspruch 2 oder 3, wobei der zusätzliche bzw. einer, mehrere oder sämtliche der zusätzlichen Riechstoffe eine Molmasse im Bereich von 150 bis 285 g/mol hat bzw. haben, vorzugsweise von 210 g/mol oder weniger.

5. Riechstoffmischung nach einem der Ansprüche 2 bis 4, vorzugsweise Parfümöl, wobei das Verhältnis der Gesamtmasse an nicht Oxacyclohexadecen-2-on und Oxacycloheptadec-10-en-2-on entsprechenden Riechstoffen zur Gesamtmasse an Oxacyclohexadecen-2-on und Oxacycloheptadec-10-en-2-on größer oder gleich 80 : 20 ist, bevorzugt größer oder gleich 90 : 10, besonders bevorzugt größer oder gleich 95 : 5.

6. Mischung nach einem der vorangehenden Ansprüche, herstellbar durch ein Verfahren umfassend folgende Schritte:
(i) Bereitstellen einer Ausgangsmischung enthaltend oder bestehend aus
trans- und cis-konfiguriertem Oxacyclohexadecen-2-on, wobei das Gewichtsverhältnis von der Verbindung (**E**) zu der Verbindung (**Z**) in der Ausgangsmischung größer als 60 : 40 ist,
oder
trans- und cis-konfiguriertem Oxacycloheptadec-10-en-2-on, wobei das Gewichtsverhältnis von der Verbindung **(E)** zu der Verbindung **(Z)** in der Ausgangsmischung größer als 60 : 40 ist,
(ii) Durchführen einer säurekatalysierten Isomerisierung, vorzugsweise unter Verwendung von Salpetersäure, der in der Ausgangsmischung enthaltenen Verbindung **(E),** so dass eine Mischung erhalten wird, worin das Gewichtsverhältnis von der Verbindung **(E)** zu der Verbindung **(Z)** in der Mischung im Bereich von 10 : 90 bis 60 : 40 liegt.

7. Verfahren zur Herstellung einer Mischung enthaltend oder bestehend aus
trans- und cis-konfiguriertem Oxacyclohexadecen-2-on, wobei das Gewichtsverhältnis von der Verbindung **(E)** zu der Verbindung **(Z)** im Bereich von 10 : 90 bis 60 : 40 liegt,
oder
aus trans- und cis-konfiguriertem Oxacycloheptadec-10-en-2-on, wobei das Gewichtsverhältnis von der Verbindung **(E)** zu der Verbindung **(Z)** im Bereich von 10 : 90 bis 60 : 40 liegt, umfassend folgende Schritte:
(i) Bereitstellen einer Ausgangsmischung enthaltend oder bestehend aus trans- und cis-konfiguriertem Oxacyclohexadecen-2-on, wobei das Gewichtsverhältnis von der Verbindung **(E)** zu der Verbindung **(Z)** in der Ausgangsmischung größer als 60 : 40 ist,
oder
aus trans- und cis-konfiguriertem Oxacycloheptadec-10-en-2-on, wobei das Gewichtsverhältnis von der Verbindung **(E)** zu der Verbindung **(Z)** in der Ausgangsmischung größer als 60 : 40 ist,
(ii) Durchführen einer säurekatalysierten Isomerisierung, vorzugsweise unter Verwendung von Salpetersäure, der in der Ausgangsmischung enthaltenen Verbindung **(E),** so dass eine Mischung erhalten wird, worin das Gewichtsverhältnis von der Verbindung **(E)** zu der Verbindung **(Z)** in der Mischung im Bereich von 10 : 90 bis 60 : 40 liegt.

8. Riechstoffmischung nach einem der Ansprüche 2 bis 6, wobei die Menge an Mischung nach Anspruch 1 bzw. die Menge an Verbindung **(E)** und Verbindung **(Z)** ausreicht,
(a) um den bzw. einen oder mehrere unangenehme Geruchseindrücke eines anderen Riechstoffs in der Riechstoffmischung zu maskieren oder zu vermindern,
und/oder
(b) um den bzw. einen oder mehrere angenehme Geruchseindrücke eines anderen Riechstoffs in der Riechstoffmischung zu verstärken.

9. Riechstoffmischung nach einem der Ansprüche 2 bis 6 oder 8, vorzugsweise Parfümöl, wobei die Gesamtmenge an Oxacyclohexadecen-2-on und Oxacycloheptadec-10-en-2-on bezogen auf das Gesamtgewicht der Riechstoffmischung 1 Gew.-% oder weniger beträgt, vorzugsweise 0,1 Gew.-% oder weniger, besonders bevorzugt 0,001 Gew.-% oder weniger.

10. Parfümiertes Produkt, enthaltend eine Mischung nach Anspruch 1 oder, vorzugsweise, eine Riechstoffmischung nach einem der Ansprüche 2 bis 6, 8 oder 9, vorzugsweise ein Parfümöl, in einer sensorisch wirksamen Menge, wobei der Anteil der Mischung bzw. der Riechstoffmischung bezogen auf das Gesamtgewicht des Produkts vorzugsweise im Bereich von 0,01, bevorzugt 0,1, bis 20, bevorzugt 10 Gew.-%, bevorzugt im Bereich von 0,1 bis 5 Gew.-%, besonders bevorzugt im Bereich von 0,25 bis 3 Gew.-%, liegt.

11. Parfümiertes Produkt nach Anspruch 10, wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und - lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

12. Verfahren zum Herstellen eines parfümierten Produkts, insbesondere eines parfümierten Produkts nach einem der Ansprüche 10 oder 11, umfassend folgende Schritte:
i) Bereitstellen einer Mischung nach Anspruch 1 oder einer Riechstoffmischung nach einem der Ansprüche 2 bis 6, 8 oder 9 oder einer ersten Verbindung **(E)** und einer zweiten Verbindung **(Z)** wie in Anspruch 1 definiert in einem Gewichtsverhältnis wie in Anspruch 1 definiert,
ii) Bereitstellen eines oder mehrerer weiterer Bestandteile des herzustellenden parfümierten Produkts, und
iii) Inkontaktbringen oder Mischen der in Schritt ii) bereitgestellten weiteren Bestandteile mit einer sensorisch wirksamen Menge der in Schritt i) bereitgestellten Bestandteile.

13. Verwendung einer Mischung oder Riechstoffmischung nach einem der Ansprüche 1 bis 6, 8 oder 9 als Geruchsmodifizierer, vorzugsweise
(a) zum Maskieren oder Vermindern des bzw. eines oder mehrerer unangenehmer Geruchseindrücke eines oder mehrerer unangenehm riechender Stoffe,
und/oder
(b) zum Verstärken des bzw. eines oder mehrerer angenehmer Geruchseindrücke eines oder mehrerer angenehm riechender Stoffe.

14. Verwendung nach Anspruch 13 in einer Zusammensetzung, vorzugsweise einem Parfümöl, die bzw. das einen oder mehrere (weitere) angenehm und/oder unangenehm riechende Stoffe enthält, deren unangenehmer Geruchseindruck durch die Mischung nach Anspruch 1 maskiert oder vermindert und/oder deren angenehmer Geruchseindruck durch die Mischung nach Anspruch 1 verstärkt wird, wobei diese(r) angenehm und/oder unangenehm riechende Stoff bzw. einer, mehrere oder sämtliche dieser angenehm und/oder unangenehm riechende Stoffe
- ausgewählt ist bzw. sind aus der Gruppe bestehend aus Alkoholen, Aldehyden, Ketonen, Ethern, Estern und Carboxylaten, vorzugsweise Aldehyden und Alkoholen,
und/oder
- eine Molmasse im Bereich von 150 bis 285 g/mol hat bzw. haben.

## Claims

1. Mixture comprising or consisting of
trans- and cis-configurated oxacyclohexadecen-2-on, wherein the weight ratio of compound **(E)** to compound **(Z)** is in the range of 10 : 90 to 60 : 40,
or
of trans- and cis-configurated oxacycloheptadec-10-en-2-on, wherein the weight ratio of compound **(E)** to compound **(Z)** is in the range of 10 : 90 to 60 : 40.

2. Mixture according to claim 1, wherein the mixture is a fragrance substance mixture, preferably a perfume oil, and furthermore preferably comprises one or more additional fragrance substances.

3. Mixture according to claim 2, wherein the additional or one, several or all of the additional fragrance substances is or are selected from the group consisting of alcohols, aldehydes, ketones, ethers, esters and carboxylates, preferably aldehydes and alcohols.

4. Mixture according to claim 2 or 3, wherein the additional or one, several or all of the additional fragrance substances has or have a molar mass in the range of 150 to 285 g/mol, preferably of 210 g/mol or less.

5. Fragrance substance mixture according to any one of the claims 2 to 4, preferably perfume oil, wherein the ratio of the total mass of fragrance substances that do not correspond to oxacyclohexadecen-2-on and oxacycloheptadec-10-en-2-on to the total mass of oxacyclohexadecen-2-on and oxacycloheptadec-10-en-2-on is greater than or equal to 80 : 20, preferably greater than or equal to 90 : 10, most preferably greater than or equal to 95 : 5.

6. Mixture according to any one of the preceding claims, producible by a method comprising the following steps:
(i) Providing a base mixture comprising or consisting of trans- and cis-configurated oxacyclohexadecen-2-on, wherein the weight ratio of compound **(E)** to compound **(Z)** in the base mixture is greater than 60 : 40,
or
of trans- and cis-configurated oxacycloheptadec-10-en-2-on, wherein the weight ratio of compound **(E)** to compound **(Z)** in the base mixture is greater than 60 : 40,
(ii) performing an acid-catalysed isomerisation, preferably with the use of nitric acid, of compound **(E)** contained in the base mixture, so that a mixture is obtained, wherein the weight ratio of compound **(E)** to compound **(Z)** in the mixture is in the range of 10 : 90 to 60 : 40.

7. Method for producing a mixture comprising or consisting of
trans- and cis-configurated oxacyclohexadecen-2-on, wherein the weight ratio of compound **(E)** to compound **(Z)** is in the range of 10 : 90 to 60 : 40,
or
of trans- and cis-configurated oxacycloheptadec-10-en-2-on, wherein the weight ratio of compound **(E) to** compound **(Z)** is in the range of 10 : 90 to 60 : 40, comprising the following steps:
(i) Providing a base mixture comprising or consisting of
trans- and cis-configurated oxacyclohexadecen-2-on, wherein the weight ratio of compound **(E)** to compound **(Z)** in the base mixture is greater than 60 : 40,
or
of trans- and cis-configurated oxacycloheptadec-10-en-2-on, wherein the weight ratio of compound **(E)** to compound **(Z)** in the base mixture is greater than 60 : 40,
(ii) performing an acid-catalysed isomerisation, preferably with the use of nitric acid, of compound **(E)** contained in the base mixture, so that a mixture is obtained, wherein the weight ratio of compound **(E)** to compound **(Z)** in the mixture is in the range of 10 : 90 to 60 : 40.

8. Fragrance substance mixture according to any one of the claims 2 to 6, wherein the amount of mixture according to claim 1 or the amount of compound **(E)** and compound **(Z)** is sufficient
(a) to mask or reduce the or one or more unpleasant olfactory impressions of another fragrance substance in the fragrance substance mixture,
and/or
(b) to enhance the or one or more pleasant olfactory impressions of another fragrance substance in the fragrance substance mixture.

9. Fragrance substance mixture according to any one of the claims 2 to 6 or 8, preferably perfume oil, wherein the total amount of oxacyclohexadecen-2-on and oxacycloheptadec-10-en-2-on with regard to the total weight of the fragrance substance mixture is 1 wt.% or less, preferably 0.1 wt.% or less, particularly preferably 0.001 wt.% or less.

10. Perfumed product, comprising a mixture according to claim 1 or, preferably, a fragrance substance mixture according to any one of the claims 2 to 6, 8 or 9, preferably a perfume oil, in a sensorially effective amount, wherein the proportion of the mixture or of the fragrance substance mixture with regard to the total weight of the product preferably is in the range of 0.01, preferably 0.1, to 20, preferably 10 wt.%, preferably in the range of 0.1 to 5 wt.%, particularly preferably in the range of 0.25 to 3 wt.%.

11. Perfumed product according to claim 10, wherein the product is selected from the group consisting of perfume extraits, eau de parfums, eau de toilettes, aftershaves, eau de colognes, pre-shave products, splash colognes, perfumed refreshing tissues, acidic, alkaline and neutral cleaning agents, textile fresheners, ironing aids, liquid detergents, powdery detergents, laundry pretreatment agents, fabric softeners, laundry soaps, laundry tablets, disinfectants, surface disinfectants, air improvers, aerosol sprays, waxes and polishes, personal care agents, hand creams and lotions, foot creams and lotions, depilatory creams and lotions, aftershave creams and lotions, tanning creams and lotions, hair care products, deodorants and antiperspirants, products for decorative cosmetic, candles, lamp oils, incense sticks, insecticides, repellents and fuels.

12. Method for producing a perfumed product, particularly a perfumed product according to any one of the claims 10 or 11, comprising the following steps:
i) Providing a mixture according to claim 1 or a fragrance substance mixture according to any one of the claims 2 to 6, 8 or 9 or a first compound **(E)** and a second compound **(Z)** as defined in claim 1, in a weight ratio as defined in claim 1,
ii) providing one or more further components of the perfumed product to be produced, and
iii) contacting or mixing the further components provided in step ii) with a sensorially effective amount of the components provided in step i).

13. Use of a mixture or of a fragrance substance mixture according to any one of the claims 1 to 6, 8 or 9 as an odor modifier, preferably
(a) for masking or reducing the or one or more unpleasant olfactory impressions of one or more unpleasantly smelling substances,
and/or
(b) for enhancing the or one or more pleasant olfactory impressions of one or more pleasantly smelling substances.

14. Use according to claim 13 in a composition, preferably in a perfume oil, that comprises one or more (further) pleasantly and/or unpleasantly smelling substances, whose unpleasant olfactory impression is masked or reduced by the mixture according to claim 1 and/or whose pleasant olfactory impression is enhanced by the mixture according to claim 1, wherein this/these pleasantly and/or unpleasantly smelling substance or one, several or all of these pleasantly and/or unpleasantly smelling substances
- is or are selected from the group consisting of alcohols, aldehydes, ketones, ethers, esters and carboxylates, preferably aldehydes and alcohols,
and/or
- has or have a molar mass in the range of 150 to 285 g/mol.

## Revendications

1. Mélange contenant ou consistant en
une oxacyclohexadécén-2-one configurée en trans et cis, dans lequel le rapport des masses du composé **(E)** au comp#osé **(Z)** se situe dans la plage de 10:90 à 60:40,
ou
une oxacycloheptadéc-10-én-2-one configurée en trans et cis, dans lequel le rapport des masses du composé **(E)** au composé **(Z)** se situe dans la plage de 10:90 à 60:40.

2. Mélange selon la revendication 1, dans lequel le mélange est un mélange de substances odorantes, de préférence une essence de parfum, et contient en outre de préférence une ou plusieurs substances odorantes supplémentaires.

3. Mélange selon la revendication 2, dans lequel la substance odorante supplémentaire ou une, plusieurs ou la totalité des substances odorantes supplémentaires est ou sont choisies dans le groupe constitué par les alcools, les aldéhydes, les cétones, les éthers, les esters et les carboxylates, de préférence les aldéhydes et les alcools.

4. Mélange selon la revendication 2 ou 3, dans lequel la substance odorante supplémentaire ou une, plusieurs ou la totalité des substances odorantes supplémentaires a ou ont une masse molaire dans la plage de 150 à 285 g/mol, de préférence de 210 g/mol ou moins.

5. Mélange de substances odorantes selon l'une quelconque des revendications 2 à 4, de préférence essence de parfum, dans lequel le rapport de la masse totale de substances odorantes ne correspondant pas à l'oxacyclohexadécén-2-one et à l'oxacy-cloheptadéc-10-én-2-one à la masse totale d'oxacyclohexadécén-2-one et d'oxacy-cloheptadéc-10-én-2-one est supérieur ou égal à 80:20, de préférence supérieur ou égal à 90:10, de manière particulièrement préférée supérieur ou égal à 95:5.

6. Mélange selon l'une quelconque des revendications précédentes, pouvant être produit par un procédé comprenant les étapes suivantes :
(i) fournir un mélange initial contenant ou consistant en
une oxacyclohexadécén-2-one configurée en trans et cis, dans lequel le rapport des masses du composé **(E)** au composé **(Z)** dans le mélange initial est supérieur à 60:40,
ou
une oxacycloheptadéc-10-én-2-one configurée en trans et cis, dans lequel le rapport des masses du composé **(E)** au composé **(Z)** dans le mélange initial est supérieur à 60:40,
(ii) réaliser une isomérisation par catalyse acide, de préférence par utilisation d'acide nitrique, du composé **(E)** contenu dans le mélange initial, de manière à obtenir un mélange dans lequel le rapport des masses du composé **(E)** au composé **(Z)** dans le mélange se situe dans la plage de 10:90 à 60:40.

7. Procédé pour fabriquer un mélange contenant ou consistant en
une oxacyclohexadécén-2-one configurée en trans et cis, dans lequel le rapport des masses du composé **(E)** au composé **(Z)** se situe dans la plage de 10:90 à 60:40,
ou
une oxacycloheptadéc-10-én-2-one configuré en trans et cis, dans lequel le rapport des masses du composé **(E)** au composé **(Z)** se situe dans la plage de 10:90 à 60:40, comprenant les étapes suivantes :
(i) fournir un mélange initial contenant ou consistant en
une oxacyclohexadécén-2-one configurée en trans et cis, dans lequel le rapport des masses du composé **(E)** au composé **(Z)** dans le mélange initial est supérieur à 60:40,
ou
une oxacycloheptadéc-10-én-2-one configurée en trans et cis, dans lequel le rapport des masses du composé **(E)** au composé **(Z)** dans le mélange initial est supérieur à 60:40,
(ii) réaliser une isomérisation par catalyse acide, de préférence par utilisation d'acide nitrique, du composé **(E)** contenu dans le mélange initial, de manière à obtenir un mélange dans lequel le rapport des masses du composé **(E)** au composé **(Z)** dans le mélange se situe dans la plage de 10:90 à 60:40.

8. Mélange de substances odorantes selon l'une quelconque des revendications 2 à 6, dans lequel la quantité de mélange selon la revendication 1 ou la quantité de composé **(E)** et de composé **(Z)** est suffisante,
(a) pour masquer ou réduire la ou une ou plusieurs impressions olfactives désagréables d'une autre substance odorante dans le mélange de substances odorantes,
et/ou
(b) pour renforcer la ou une ou plusieurs impressions olfactives agréables d'une autre substance odorante dans le mélange de substances odorantes.

9. Mélange de substances odorantes selon l'une quelconque des revendications 2 à 6 ou 8, de préférence essence de parfum, dans lequel la quantité totale d'oxacyclo-hexadécén-2-one et d'oxacycloheptadéc-10-én-2-one par rapport au poids total du mélange de substances odorantes est de 1 % en poids ou moins, de préférence de 0,1 % en poids ou moins, de manière particulièrement préférée de 0,001 % en poids ou moins.

10. Produit parfumé, contenant un mélange selon la revendication 1 ou, de préférence, un mélange de substances odorantes selon l'une quelconque des revendications 2 à 6, 8 ou 9, de préférence une essence de parfum, en une quantité sensoriellement efficace, dans lequel la part du mélange ou du mélange de substances odorantes par rapport au poids total du produit se situe de préférence autour de 0,01, de préférence de 0,1 à 20, de préférence de 10 % en poids, de préférence dans la plage de 0,1 à 5 % en poids, de manière particulièrement préférée dans la plage de 0,25 à 3 % en poids.

11. Produit parfumé selon la revendication 10, dans lequel le produit est choisi dans le groupe constitué par les extraits de parfum, les eaux de parfum, les eaux de toilette, les eaux de rasage, les eaux de Cologne, les produits de pré-rasage, les eaux de Cologne splash, les lingettes rafraîchissantes parfumées, les produits de nettoyage acides, alcalins et neutres, les produits de rafraîchissement des textiles, les aides au repassage, les produits de lessive liquides, les produits de lessive en poudre, les produits de prétraitement du linge, les adoucissants pour le linge, les savons de lavage, les pastilles de lavage, les désinfectants, les désinfectants de surface, les améliorateurs d'air, les atomiseurs d'aérosols, les cires et produits lustrants, les produits de soins corporels, les crèmes et lotions pour les mains, les crèmes et lotions pour les pieds, les crèmes et lotions dépilatoires, les crèmes et lotions après-rasage, les crèmes et lotions bronzantes, les produits de soins capillaires, les déodorants et antiperspirants, les produits de cosmétique décorative, les bougies, les huiles pour lampes, les bâtonnets d'encens, les insecticides, les répulsifs et les carburants.

12. Procédé pour fabriquer un produit parfumé, en particulier un produit parfumé selon l'une quelconque des revendications 10 ou 11, comprenant les étapes suivantes :
i) fournir un mélange selon la revendication 1 ou un mélange de substances odorantes selon l'une quelconque des revendications 2 à 6, 8 ou 9, ou un premier composé (E) et un deuxième composé (Z) tels que définis dans la revendication 1, dans un rapport de masses tel que défini dans la revendication 1,
ii) fournir un ou plusieurs composants supplémentaires du produit parfumé à fabriquer, et
iii) mettre en contact ou mélanger les composants supplémentaires fournis à l'étape (ii) avec une quantité sensoriellement efficace des composants fournis à l'étape i).

13. Utilisation d'un mélange ou d'un mélange de substances odorantes selon l'une quelconque des revendications 1 à 6, 8 ou 9 en tant que modificateur d'odeur, de préférence
(a) pour masquer ou réduire la ou une ou plusieurs impressions olfactives désagréables d'une ou plusieurs substances à l'odeur désagréable,
et/ou
(b) pour renforcer la ou une ou plusieurs impressions olfactives agréables d'une ou plusieurs substances à l'odeur agréable.

14. Utilisation selon la revendication 13 dans une composition, de préférence une essence de parfum, qui contient une ou plusieurs (autres) substances à odeur agréable et/ou désagréable, dont l'impression olfactive désagréable est masquée ou réduite par le mélange selon la revendication 1 et/ou dont l'impression olfactive agréable est renforcée par le mélange selon la revendication 1, dans laquelle cette ou ces substances à odeur agréable et/ou désagréable ou une, plusieurs ou la totalité de ces substances à odeur agréable et/ou désagréable
- sont choisies dans le groupe constitué par les alcools, les aldéhydes, les cétones, les éthers, les esters et les carboxylates, de préférence les aldéhydes et les alcools,
et/ou
- ont une masse molaire dans la plage de 150 à 285 g/mol.
